Europäisches Patentamt

European Patent Office

Office européen des brevets

(19)

(11) Veröffentlichungsnummer: **0 202 365**

A2

(12) **EUROPÄISCHE PATENTANMELDUNG**

(21) Anmeldenummer: 85200873.9

(22) Anmeldetag: 04.06.85

(51) Int. Cl.⁴: **A 61 K 47/00**
**A 61 K 31/48**

(30) Priorität: 13.05.85 AT 1439/85

(43) Veröffentlichungstag der Anmeldung:
26.11.86 Patentblatt 86/48

(84) Benannte Vertragsstaaten:
AT CH DE LI

(71) Anmelder: F. Joh. Kwizda Unternehmens-
Verwaltungsgesellschaft m.b.H.
Dr. Karl Lueger-Ring 6
A-1011 Wien(AT)

(72) Erfinder: Kubjacek, Manfred, Dipl.-Ing. Dr.
Floridsdorfer Hauptstrasse 35/3/12
A-1210 Wien(AT)

(74) Vertreter: Müllner, Erwin
Patentanwälte Dr. Erwin Müllner, Dipl.-Ing. Armin Häupl,
Dipl.-Ing. Werner Katschinka Weihburggasse 9
A-1010 Wien(AT)

(54) **Verfahren zur Herstellung stabiler flüssiger Lösungen von Ergolinderivaten.**

(57) Die Erfindung betrifft stabile Lösungen von Ergolinderivaten in Lösungsmittelgemischen aus 45-55 Gew.-% Wasser,
1-5 Gew.-% Ethanol und 4-54 Gew.-% Propylenglykol-1,2,
wobei die Lösungen einen pH-Wert von 4,0 bis 5,0 aufweisen.

EP 0 202 365 A2

Croydon Printing Company Ltd.

- 1 -

## Verfahren zur Herstellung stabiler flüssiger Lösungen von Ergolinderivaten

Die Erfindung betrifft ein Verfahren zur Herstellung stabiler flüssiger Lösungen von Ergolinderivaten, nämlich Dihydroergocornin oder Dihydroergocryptin oder Dihydroergocristin oder Mischungen dieser Wirkstoffe oder der Salze dieser Wirkstoffe mit unbedenklichen Säuren in einer Wirkstoffkonzentration von 0,1 bis 0,5 Gew.-%.

Die Instabilität der genannten Alkaloide, besonders in Tropflösungen, führt zu erheblichen Schwierigkeiten bei der Herstellung pharmazeutisch brauchbarer flüssiger Darreichungsformen, da es im Verlauf der Lagerung zu Wirkstoffverlusten infolge von Umlagerungs- und Oxidationsreaktionen kommt. Um diesem nachteiligen Effekt zu begegnen, wird häufig eine Begasung der Lösung mit Inertgas bei der Abfüllung in die zum Verkauf bestimmten Gefäße durchgeführt. Durch diese technisch aufwendigen Maßnahmen kann der erwünschte Zweck jedoch nur unvollständig erreicht werden, da nach Öffnung der Gefäße die Wirkstoffkonzentration abfällt.

Um diese Zersetzungserscheinungen zu verhindern, wurden verschiedene Vorschläge zur Stabilisierung solcher Lösungen gemacht. So ist bekannt, die genannten Alkaloide in einem Gemisch aus einwertigen und mehrwertigen Alkoholen, wobei der Wassergehalt maximal 40 Gew.-% beträgt, oder in einem ethylalkoholfreien Lösungsmittelgemisch aus Wasser und einem oder mehreren mehrwertigen Alkoholen zu lösen. Nachteilig an den genannten Vorschlägen sind in einem Fall die oft komplizierten Rezepturen sowie die Verwendung größerer Mengen an Ethylalkohol, was bestimmte Risikogruppen wie Leberkranke, Alkoholkranke, Epileptiker, Hirngeschädigte, Schwangere und Kinder von der Dauertherapie ausschließt, und im anderen Fall die geschmacklich nicht zufriedenstellenden Auswirkungen zu großer Konzentrationen von Propylenglykol.

Das Ziel, welches der vorliegenden Erfindung zugrunde liegt, ist daher die Auffindung eines Verfahrens, nach dem mit möglichst einfacher Rezeptur eine ethanolarme, für die orale Verabreichung geschmacklich einwandfreie Tropflösung hergestellt werden kann, die über längere Zeit, auch nach Anbruch des Behältnisses, ihren Wirkstoffgehalt unverändert beibehält.

Überraschenderweise wurde gefunden, daß stabile Lösungen auch mit einem wesentlich größeren Wassergehalt als 40 Gew.-% und einem relativ kleinen Ethanolgehalt hergestellt werden können, wenn der pH-Wert in einem optimalen Bereich gehalten wird. Dies war nach dem Stand der Technik nicht zu erwarten.

Das erfindungsgemäße Verfahren ist dadurch gekennzeichnet, daß man die Wirkstoffe in einem Gemisch aus 45 - 55 Gew.-% Wasser, 1 - 5 Gew.-% Ethanol und 40 - 54 Gew.-% Propylenglykol-1,2 auflöst, wobei der pH-Wert gegebenenfalls durch Zusatz von pharmazeutisch unbedenklichen Säuren oder Basen auf 4,0 bis 5,0 eingestellt wird.

Die erfindungsgemäß hergestellten Lösungen enthalten also 45 - 55 Gew.-% Wasser, 1 - 5 Gew.-% Ethanol und 40 -54 Gew.-% Propylenglykol (Ergänzung auf 100 %), wobei durch das gewählte Mischungsverhältnis und die Einstellung eines pH-Wert-Optimums weder Geschmackskorrigentien noch Stabilisatoren und Antioxidantien benötigt werden. Obzwar die Konzentration der hydrierten Ergotalkaloide und deren Salze in den Lösungen nicht kritisch ist, empfiehlt es sich, Lösungen mit einer Wirkstoffkonzentration von 0,1 bis 0,5 Gew.-% zu verwenden.

Kennzeichnend für die Erfindung ist weiters ein optimaler pH-Bereich von 4,0 - 5,0, auf welchen die Lösung eingestellt wird. Dieser pH-Bereich ist für die Stabilität entscheidend, da einerseits bei pH-Werten unter 4 durch Umlagerung zu Aci-Formen die Stabilität abnimmt, andererseits auch bei pH-Werten über 5 ein zunehmender Konzentrationsabfall beobachtet wurde. Die Einstellung des pH-Wertes auf den gewünschten Wert kann erforderlichenfalls z.B. mit Weinsäure oder mit Natronlauge erfolgen.

Es wurde weiter überraschend festgestellt, daß diese Lösungen ohne jegliche Schutzbegasung hergestellt und abgefüllt werden können und dennoch ihren Wirkstoffgehalt unverändert beibehalten, wenn man sie zwischen Raumtemperatur und 40°C lagert. Die erfindungsgemäßen Lösungen sind auch nach Anbruch der Tropfflasche gegen einen Wirkstoffabfall aufgrund des eindringenden Luftsauerstoffs stabiler als die herkömmlichen Troplösungen.

Das erfindungsgemäße Verfahren wird zweckmäßiger Weise durch Lösen der genannten Alkaloide in Wasser und Ethanol bei Raumtemperatur und anschließende Zumischung von Propylenglykol unter Verrühren ausgeführt. Die Verwendung einer Inertgasatmosphäre sowie der Ausschluß von Tageslicht sind im Hinblick auf die hohe Stabilität der Lösung nicht notwendig.

Die erfindungsgemäß hergestellten Lösungen sind so stabil, daß der Alkaloidgehalt nach 60-tägiger Streßlagerung bei 70°C praktisch unverändert bleibt und keine sichtbare Gelbfärbung eintritt.

Beispiel 1: 0,1 g eines Gemisches der Methansulfonatsalze von Dihydroergocristin, Dihydroergocryptin und Dihydroergocornin (1/1/1) werden in einer Mischung aus 55,0g Wasser (entmineralisiert) und 1,5 g Ethanol (96,0 % rein) unter Rühren gelöst. Dann werden 45,0 g Propylenglykol zugemischt, und die Lösung wird einer Druckfiltration unterworfen. Anschließend erfolgt die Abfüllung in Tropfflaschen; der pH-Wert der Lösung ist 4,81.

Beispiel 2: 0,4 g eines Gemisches der Methansulfonatsalze von Dihydroergocristin, Dihydroergocryptin und Dihydroergocornin (1/1/1) werden in einer Mischung aus 46,0 g Wasser (entmineralisiert) und 5,0 g Ethanol (96 % rein) unter Rühren gelöst. Danach werden 50,0 g Propylenglykol zugemischt. Nach der Druckfiltration erfolgt die Abfüllung in Tropfflaschen, der pH-Wert der Lösung ist 4,21.

Beispiel 3: 0,2 g eines Gemisches der Methansulfonatsalze von Dihydroergocristin, Dihydroergocryptin und Dihydroergocornin (1/1/1) werden in einer Mischung aus 52,2 g Wasser (entmineralisiert) und 5,0 g Ethanol (96 % rein) unter Rühren gelöst. Dann werden 45,0 g Propylenglykol zugemischt, und die Lösung wird druckfiltriert. Danach erfolgt die Abfüllung in Tropfflaschen, der pH-Wert der Lösung ist 4,44.

Beispiel 4: 0,3 g Dihydroergocristin werden in 5,0 g Ethanol aufgeschlämmt und mit 41,0 g Propylenglykol versetzt. Danach wird mit 55,0 g entmineralisiertem Wasser verdünnt. Die erhaltene  Wirkstofflösung wird mit Weinsäure auf einen pH-Wert von 4,5 eingestellt.

Patentansprüche:

1. Verfahren zur Herstellung von stabilen flüssigen Lösungen von Ergolinderivaten, nämlich Dihydroergocristin oder Dihydroergocryptin oder Dihydroergocornin oder Mischungen dieser Wirkstoffe oder der Salze dieser Wirkstoffe mit unbedenklichen Säuren, in einer Wirkstoffkonzentration von 0,1 bis 0,5 Gew.-%, dadurch gekennzeichnet, daß man die Wirkstoffe in einem Gemisch aus 45-55 Gew.-% Wasser, 1 - 5 Gew.-% Ethanol und 40 - 54 Gew.-% Propylenglykol 1,2 auflöst, wobei der pH-Wert gegebenenfalls durch Zusatz von pharmazeutisch unbedenklichen Säuren oder Basen auf 4,0 bis 5,0 eingestellt wird.

2. Verfahren nach Anspruch 1, dadurch gekennzeichnet, daß ein Gemisch aus 52,2 Gew.-Teilen Wasser, 5,0 Gew.-Teilen Ethanol und 45,0 Gew.-Teilen Propylenglykol 1,2 eingesetzt wird.